(19) **Europäisches Patentamt European Patent Office Office européen des brevets**

(11) **EP 3 332 869 B1**

(12) **FASCICULE DE BREVET EUROPEEN**

(45) Date de publication et mention
de la délivrance du brevet:
**09.12.2020 Bulletin 2020/50**

(21) Numéro de dépôt: **17306378.5**

(22) Date de dépôt: **12.10.2017**

(51) Int Cl.:
*B01J 23/44* (2006.01)     *B01J 35/00* (2006.01)
*B01J 21/04* (2006.01)     *B01J 35/10* (2006.01)
*B01J 37/02* (2006.01)     *B01J 37/10* (2006.01)
*B01J 37/18* (2006.01)     *B01J 29/76* (2006.01)
*C07C 5/05* (2006.01)     *C10G 45/40* (2006.01)
*C07C 5/09* (2006.01)

(54) **CATALYSEUR D'HYDROGENATION SELECTIVE DE COUPES C3 DE VAPOCRAQUAGE ET/OU DE CRAQUAGE CATALYTIQUE**

KATALYSATOR ZU SELEKTIVEN HYDRIERUNG VON C3-FRAKTIONEN DURCH DAMPFSPALTUNG UND/ODER KATALYTISCHER SPALTUNG

CATALYST FOR SELECTIVE HYDROGENATION OF C3 FRACTIONS FROM STEAM CRACKING AND/OR CATALYTIC CRACKING

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **29.11.2016 FR 1661621**

(43) Date de publication de la demande:
**13.06.2018 Bulletin 2018/24**

(73) Titulaire: **IFP Energies nouvelles
92852 Rueil-Malmaison Cedex (FR)**

(72) Inventeurs:
• **BOUALLEG, Malika
69100 VILLEURBANNE (FR)**
• **AVENIER, Priscilla
38000 GRENOBLE (FR)**

(74) Mandataire: **IFP Energies nouvelles
Département Propriété Industrielle
Rond Point de l'échangeur de Solaize
BP3
69360 Solaize (FR)**

(56) Documents cités:
FR-A1- 2 458 524     FR-A1- 2 991 197
FR-A5- 2 070 995     US-A1- 2005 137 433

**Description**

**Domaine technique**

Le procédé d'hydrogénation sélective permet de transformer les composés polyinsaturés des coupes pétrolières par conversion des composés les plus insaturés vers les alcènes correspondants en évitant la saturation totale et donc la formation des alcanes correspondants.

L'objet de l'invention est de proposer un catalyseur à performances améliorées ainsi qu'un mode de préparation de ce catalyseur, ce catalyseur possédant de très bonnes performances pour les procédés d'hydrogénation sélective des composés hydrocarbonés insaturés présents dans les coupes d'hydrocarbures C3 de vapocraquage et/ou de craquage catalytique.

**Etat de la technique**

Les catalyseurs d'hydrogénation sélective des coupes C3 de vapocraquage et/ou de craquage catalytique sont généralement à base de palladium, sous forme de petites particules métalliques déposées sur un support qui peut être un oxyde réfractaire. La teneur en palladium et la taille des particules de palladium font partie des critères qui ont une importance sur l'activité et la sélectivité des catalyseurs.

La répartition macroscopique des particules métalliques dans le support constitue également un critère important, principalement dans le cadre de réactions rapides et consécutives telles que les hydrogénations sélectives. Il faut généralement que ces éléments se situent dans une croûte à la périphérie du support afin d'éviter les problèmes de transfert de matière intragranulaire pouvant conduire à des défauts d'activité et une perte de sélectivité. Par exemple, le document US2006/025302 décrit un catalyseur pour l'hydrogénation sélective de l'acétylène et de dioléfines, comprenant du palladium réparti de telle façon que 90% du palladium est introduit dans le catalyseur dans une croûte inférieure à 250 μm.

Par ailleurs, afin d'améliorer la sélectivité des catalyseurs d'hydrogénation sélective, et en particulier des catalyseurs d'hydrogénation sélective des composés hydrocarbonés insaturés présents dans les coupes d'hydrocarbures C3 de vapocraquage et/ou de craquage catalytique, il a été proposé dans l'art antérieur d'ajouter un second métal choisi parmi le groupe IB, de préférence de l'argent, afin d'obtenir des catalyseurs bimétalliques palladium-Argent (Pd-Ag). De tels catalyseurs sont décrits dans les documents FR2882531 et FR2991197. L'ajout d'argent dans la phase active du catalyseur a pour effet principal de diminuer la dispersion métallique du palladium, mais ne fait pas en revanche changer la répartition en tailles des particules du catalyseur. FR2458524, FR2070995 et US2005137433 traitent également de catalyseur d'hydrogénation sélective à performances améliorées.

La Demanderesse a découvert de manière surprenante que les performances d'un catalyseur de palladium, le palladium étant en partie réparti dans une croûte en périphérie du support, pouvaient être nettement améliorées dans un procédé d'hydrogénation sélective des composés hydrocarbonés insaturés présents dans les coupes d'hydrocarbures C3 de vapocraquage et/ou de craquage catalytique lorsque ledit catalyseur a une phase active constituée uniquement de palladium, i.e. qu'il ne comprend pas de métal autre que le palladium dans la phase active, et en particulier de l'argent, ledit catalyseur présentant une dispersion métallique du palladium inférieure à 20%, avec une teneur en palladium et un support poreux de surface spécifique bien déterminés. Un tel catalyseur a pu être obtenu par un procédé de préparation comprenant une étape d'imprégnation à sec avec un précurseur de palladium bien choisi ainsi qu'une étape de traitement hydrothermal spécifique, engendrant un frittage du catalyseur, ayant pour effet de diminuer la dispersion métallique du palladium au sein du catalyseur, et permettant de manière inattendue, d'obtenir un catalyseur présentant des performances accrues en terme de sélectivité.

En effet, le catalyseur selon l'invention présente une sélectivité importante permettant une hydrogénation des composés acétyléniques et/ou alléniques et/ou dioléfines, tout en limitant l'hydrogénation totale des mono-oléfines (propane dans le cas des coupes C3), et en limitant toute réaction de polymérisation provoquant une diminution du rendement en propylène et une désactivation précoce du catalyseur.

**Objets de l'invention**

Un premier objet selon l'invention concerne un catalyseur dont la phase active est constituée de palladium, et un support poreux d'alumine dans lequel :

- la teneur en palladium dans le catalyseur est comprise entre 0,0025 et 1% poids par rapport au poids total du catalyseur ;
- au moins 80% poids du palladium est réparti dans une croûte à la périphérie du support poreux, l'épaisseur de ladite croûte étant comprise entre 25 et 500 μm ;

- la surface spécifique du support poreux est comprise entre 1 et 50 m$^2$/g ;
- la dispersion métallique D du palladium est inférieure à 20%.

De préférence, la dispersion métallique D du palladium est inférieure ou égale à 18%. Avantageusement, la teneur en palladium dans le catalyseur est comprise entre 0,025 et 0,8% poids par rapport au poids total du catalyseur.

De préférence, la surface spécifique du support poreux est comprise entre 1 et 40 m$^2$/g. Avantageusement, au moins 80% poids du palladium est réparti dans une croûte à la périphérie du support poreux, l'épaisseur de ladite croûte étant entre 50 et 450 $\mu$m.

De préférence, le support poreux est de l'alumine. Le volume total poreux du support est compris entre 0,1 et 1,5 cm$^3$/g. De préférence, le support poreux comprend entre 0,0050 et 0,25% en poids de soufre par rapport au poids total du catalyseur.

Le palladium se présente sous la forme de particules de taille moyenne comprise entre 4 à 10 nm.

Un autre objet concerne un procédé de préparation d'un catalyseur selon l'invention, comprenant les étapes suivantes :

a) on prépare une solution aqueuse comprenant au moins un sel précurseur du palladium ;
b) on imprègne ladite solution sur le support poreux d'alumine ;
c) optionnellement, on soumet le support poreux imprégné obtenu à l'étape b) à une maturation afin d'obtenir un précurseur de catalyseur ;
d) on sèche le précurseur de catalyseur obtenu à l'étape b) ou c) à une température comprise entre 70°C et 200°C pendant une durée comprise entre 0,5 et 20 h ;
e) optionnellement, on calcine le catalyseur séché obtenu à l'étape d) à une température comprise entre 250 et 900°C ;
f) on réalise un traitement hydrothermal du catalyseur séché obtenu à l'étape d) ou du catalyseur calciné obtenu à l'étape e) à une température comprise entre 600°C et 700°C, sous air, comprenant entre 300 et 4500 grammes d'eau par kg d'air ;
g) optionnellement, on soumet le catalyseur obtenu à l'issue de l'étape f) à un traitement réducteur par mise en contact avec un gaz réducteur.

Avantageusement, le sel précurseur du palladium est choisi parmi le chloropalladate de sodium ou le nitrate de palladium.

Avantageusement, à l'étape b), on imprègne ladite solution sur un support poreux par imprégnation à sec.

A l'étape f), on réalise un traitement hydrothermal du catalyseur séché obtenu à l'étape d) ou du catalyseur calciné obtenu à l'étape e) à une température comprise entre 600°C et 700°C, sous air, comprenant entre 300 et 4500 grammes d'eau par kg d'air. Encore un autre objet selon l'invention concerne un procédé d'hydrogénation sélective comprenant la mise en contact d'une coupe C3 de vapocraquage et/ou de craquage catalytique avec le catalyseur selon l'invention ou préparé selon l'invention, dans lequel la température est comprise entre 0 et 300°C, à une pression comprise entre 0,1 et 10 MPa, à un ratio molaire hydrogène/(composés polyinsaturés à hydrogéner) entre 0,1 et 10 et à une vitesse volumique horaire V.V.H. comprise entre 0,1 et 200 h$^{-1}$ pour un procédé réalisé en phase liquide, à un ratio molaire hydrogène/(composés polyinsaturés à hydrogéner) entre 0,5 et 1000 et à une vitesse volumique horaire V.V.H. entre 100 et 40000 h$^{-1}$ pour un procédé réalisé en phase gazeuse.

**Description détaillée de l'invention**

Dans la suite, les groupes d'éléments chimiques sont donnés selon la classification CAS (CRC Handbook of Chemistry and Physics, éditeur CRC press, rédacteur en chef D.R. Lide, 81ème édition, 2000-2001). Par exemple, le groupe IB selon la classification CAS correspond aux métaux de la colonne 11 selon la nouvelle classification IUPAC.

1. <u>Définitions</u>

<u>Dispersion métallique des particules (D)</u>

La dispersion de particules est un nombre sans unité, souvent exprimé en %. La dispersion est d'autant plus grande que les particules sont petites. Elle est définie dans la publication de R. Van Hardeveld et F. Hartog, « The statistics of surface atoms and surface sites on metal crystals», Surface Science 15, 1969, 189-230.

Définition de l'épaisseur de croûte de palladium

Afin d'analyser la répartition de la phase métallique sur le support, on mesure une épaisseur de croûte par microsonde de Castaing (ou microanalyse par microsonde électronique). L'appareil utilisé est un CAMECA XS100, équipé de quatre cristaux monochromateurs permettant l'analyse simultanée de quatre éléments. La technique d'analyse par microsonde de Castaing consiste en la détection de rayonnement X émis par un solide après excitation de ses éléments par un faisceau d'électrons de hautes énergies. Pour les besoins de cette caractérisation, les grains de catalyseur sont enrobés dans des plots de résine époxy. Ces plots sont polis jusqu'à atteindre la coupe au diamètre des billes ou extrudés puis métallisés par dépôt de carbone en évaporateur métallique. La sonde électronique est balayée le long du diamètre de cinq billes ou extrudés pour obtenir le profil de répartition moyen des éléments constitutifs des solides.

Lorsque le palladium est reparti en croûte, sa concentration locale diminue généralement progressivement lorsqu'elle est mesurée en partant du bord du grain catalytique vers l'intérieur. Une distance du bord du grain, à laquelle la teneur locale en palladium devient nulle, ne peut souvent pas être déterminée avec précision et reproductibilité. Afin de mesurer une épaisseur de croûte qui est significative pour la majorité des particules de palladium, l'épaisseur de croûte est définie comme la distance au bord du grain contenant 80% en poids de palladium.

Elle est définit dans la publication de L. Sorbier et al. "Measurement of palladium crust thickness on catalyst by EPMA" Materials Science and Engineering 32 (2012). A partir du profil de répartition obtenu par la microsonde de Castaing (c(x)), on peut calculer la quantité cumulée $Q(y)$ de palladium dans le grain en fonction de la distance y au bord du grain de rayon r.

Pour une bille (i.e. pour des grains de catalyseur non conforme à l'invention):

Pour une bille (i.e. pour des grains de catalyseur non conforme à l'invention):

$$Q(y) = \int_{-r}^{-y} c(x)4\pi.x^2 dx + \int_{y}^{r} c(x)4\pi.x^2 dx$$

Pour un extrudé :

$$Q(y) = \int_{-r}^{-r+y} c(x)2\pi.x dx + \int_{r-y}^{r} c(x)2\pi.x dx$$

avec

r : rayon du grain ;
y : distance au bord du grain ;
x : variable d'intégration (position sur le profil).

On suppose que le profil de concentration suit le diamètre pris de x = - r à x = + r (x = 0 étant le centre).

$Q(r)$ correspond ainsi à la quantité totale de l'élément dans le grain. On résout ensuite numériquement l'équation suivante en y :

$$\frac{Q(y)}{Q(r)} = 0{,}8$$

c étant une fonction strictement positive, Q est donc une fonction strictement croissante et cette équation possède une seule solution qui est l'épaisseur de croûte.

2. Catalyseur

L'invention concerne un catalyseur comprenant une phase active constituée de palladium, et un support poreux comprenant au moins un oxyde réfractaire sélectionné dans le groupe constitué par la silice, l'alumine et la silice-alumine, dans lequel :

- la teneur en palladium dans le catalyseur est comprise entre 0,0025 et 1% poids par rapport au poids total du catalyseur, de façon préférée entre 0,025 et 0,8 % poids ;
- au moins 80% poids du palladium est réparti dans une croûte à la périphérie du support poreux, l'épaisseur de ladite croûte étant comprise entre 25 et 500 $\mu$m, de préférence entre 50 et 450 $\mu$m, plus préférentiellement entre 100 et 400 $\mu$m, et encore plus préférentiellement entre 100 et 350 $\mu$m ;
- la surface spécifique du support poreux est comprise entre 1 et 50 m$^2$/g, de manière préférée entre 1 et 40 m$^2$/g, et encore plus préférentiellement entre 1 et 30 m$^2$/g, et de manière encore plus préférée entre 1 et 25 m$^2$/g ;
- la dispersion métallique D du palladium est inférieure à 20 %, de préférence inférieure ou égale à 18%.

La taille moyenne des particules de palladium est comprise entre 4 à 10 nm, de préférence entre 3 et 6 nm. La taille moyenne des cristallites est déduite des mesures de dispersion métallique des particules (D), en appliquant les relations dispersion-taille de particules connues de l'homme du métier et décrites dans "Analyse physico-chimique des catalyseurs industriels, Chapitre I, Éditions Technip, Paris 2001".

Le support poreux est de l'alumine. L'alumine peut être présente sous toutes les formes cristallographiques possibles : alpha, delta, téta, chi, gamma, etc., prises seules ou en mélange. De manière très préférée, on choisit l'alumine alpha-téta.

La surface spécifique du support poreux est comprise entre 1 et 50 m$^2$/g, de manière préférée entre 1 et 40 m$^2$/g, et encore plus préférentiellement entre 1 et 30 m$^2$/g, et de manière encore plus préférée entre 1 et 25 m$^2$/g. La surface spécifique BET est mesurée par physisorption à l'azote. La surface spécifique BET est mesurée par physisorption à l'azote selon la norme ASTM D3663-03 tel que décrit dans Rouquerol F.; Rouquerol J.; Singh K. « Adsorption by Powders & Porous Solids: Principle, methodology and applications », Academic Press, 1999.

Le volume total poreux du support est compris entre 0,1 et 1,5 cm$^3$/g, de préférence compris entre 0,2 et 1,4 cm$^3$/g, et encore plus préférentiellement compris entre 0,25 et 1,3 cm$^3$/g. Le volume poreux total est mesuré par porosimétrie au mercure selon la norme ASTM D4284-92 avec un angle de mouillage de 140°, par exemple au moyen d'un appareil modèle Autopore® III de la marque Microméritics®.

Dans un mode de réalisation selon l'invention, le support du catalyseur d'hydrogénation sélective est purement mésoporeux, i.e. qu'il présente un diamètre de pores compris entre 2 et 50 nm, de préférence entre 5 et 30 nm et de manière encore préférée de 8 à 20 nm.

Dans un autre mode de réalisation selon l'invention, le support du catalyseur d'hydrogénation sélective est bimodal, le premier mode étant mésoporeux, i.e. qu'il présente un diamètre de pores compris entre 2 et 50 nm, de préférence entre 5 et 30 nm et de manière encore préférée de 8 à 20 nm, et le second macroporeux, i.e. qu'il présente des pores de diamètre supérieur à 50 nm. Ledit support présente avantageusement un volume poreux des pores ayant un diamètre de pores compris entre 50 et 700 nm inférieur à 20 % du volume poreux total du support, de préférence inférieur à 18 % du volume poreux total du support et de manière particulièrement préférée inférieur à 15 % du volume poreux total du support.

Le support peut comprendre éventuellement du soufre. La teneur en soufre comprise dans le support peut être comprise entre 0,0050 et 0,25% en poids par rapport au poids total du catalyseur, de préférence entre 0,0075 et 0,20% en poids.

Selon l'invention, le support poreux se présente sous forme de billes, de trilobes, d'extrudés, de pastilles, ou d'agglomérats irréguliers et non sphériques dont la forme spécifique peut résulter d'une étape de concassage. De manière très avantageuse, le support se présente sous forme de billes ou d'extrudés. De manière encore plus avantageuse, le support se présente sous forme de billes. Le diamètre des billes est comprise entre 1 mm et 10 mm, de préférence entre 2 et 8 mm, et encore plus préférentiellement entre 2 et 6 mm.

## 3. Procédé de préparation

L'invention concerne également un procédé de préparation du catalyseur. Le dépôt de la solution de palladium sur le support peut être réalisé selon toutes les techniques connues de l'homme du métier. De préférence, la solution de palladium est déposée par méthode d'imprégnation à sec.

Plus particulièrement, le procédé de préparation du catalyseur selon l'invention comprend d'une manière générale les étapes suivantes :

- une étape a) de préparation d'une solution aqueuse comprenant au moins un sel précurseur du palladium ;
- une étape b) de préparation d'un support imprégné par imprégnation de ladite suspension obtenue à l'étape a) avec un support poreux comprenant au moins un oxyde réfractaire sélectionné dans le groupe constitué par la silice, l'alumine et la silice-alumine ;
- optionnellement, une étape c) de maturation dudit support imprégné ;
- une étape d) de séchage du précurseur de catalyseur obtenu à l'étape b) ou c) ;

- optionnellement, une étape e) de calcination du catalyseur séché obtenu à l'étape d) à une température comprise entre 250 et 900°C ;
- une étape f) de traitement hydrothermal du catalyseur séché obtenu à l'étape d) ou du catalyseur calciné obtenu à l'étape e), à une température comprise entre 600°C et 700°C, sous air, de préférence sus air de combustion, comprenant entre 300 et 4500 00 et 4500 grammes d'eau par kg d'air ;
- optionnellement, une étape g) de traitement réducteur par mise en contact avec un gaz réducteur.

Les différentes étapes sont explicitées en détail ci-après.

a) préparation d'une solution aqueuse comprenant au moins un sel précurseur du palladium

Lors de l'étape a), on prépare une solution aqueuse comprenant au moins un sel précurseur du palladium. Le sel précurseur du palladium est de préférence sélectionné parmi le chloropalladate de sodium et le nitrate de palladium.

b) préparation d'un support imprégné

Le dépôt du palladium sur le support poreux peut être réalisé par imprégnation, à sec ou en excès, de la solution aqueuse obtenue à l'étape a) sur un support de surface spécifique comprise entre 1 et 50 $m^2/g$, de manière préférée entre 1 et 40 $m^2/g$, et encore plus préférentiellement entre 1 et 30 $m^2/g$, et de manière encore plus préférée entre 1 et 25 $m^2/g$, le volume de ladite solution aqueuse étant compris entre 0,9 et 1,1 fois le volume poreux du support.
La mise en contact de la solution d'imprégnation avec le support peut être réalisée par imprégnation à sec ou par imprégnation en excès, en mode statique ou dynamique.

c) maturation du support imprégné lors de l'étape b) (étape optionnelle)

Après imprégnation, le support imprégné est généralement maturé à l'état humide pendant 0,5 à 40 heures, de manière préférée pendant 1 à 30 heures. Des durées plus longues ne sont pas exclues, mais n'apportent pas nécessairement d'amélioration.

d) séchage du précurseur de catalyseur obtenu à l'étape b) ou c)

Le précurseur du catalyseur est généralement séché afin d'éliminer toute ou une partie de l'eau introduite lors de l'imprégnation, de préférence à une température comprise entre 50°C et 250°C, de manière plus préférée entre 70°C et 200°C. La durée du séchage est comprise entre 0,5 h et 20 h. Des durées plus longues ne sont pas exclues, mais n'apportent pas nécessairement d'amélioration.
Le séchage est généralement effectué sous air de combustion d'un hydrocarbure, de préférence du méthane, ou sous air chauffé comprenant entre 0 et 80 grammes d'eau par kilogramme d'air de combustion, un taux d'oxygène compris entre 5% et 25% volume et un taux de dioxyde de carbone compris entre 0% et 10% volume.

e) calcination sous air de combustion du catalyseur séché obtenu à l'étape d) (étape optionnelle)

Après séchage, le catalyseur peut être calciné sous air, de préférence de combustion, et plus préférentiellement un air de combustion du méthane, comprenant entre 40 et 80 gramme d'eau par kg d'air, un taux d'oxygène compris entre 5% et 15% volume et un taux de $CO_2$ compris entre 4% et 10% volume. La température de calcination est généralement comprise entre 250°C et 900°C, de préférence comprise entre environ 300°C et environ 500°C. La durée de calcination est généralement comprise entre 0,5 h et 5 h.

f) traitement hydrothermal du catalyseur séché obtenu à l'étape d) ou calciné obtenu à l'étape e)

Après l'étape d) de séchage ou l'étape e) de calcination, le catalyseur subit un traitement hydrothermal sous air, de préférence sous air de combustion, comprenant entre 300 et 4500 grammes d'eau par kg d'air, de préférence entre 500 et 4000 grammes d'eau par kg d'air. La température du traitement hydrothermal est comprise entre 600°C et 700°C. La durée du traitement hydrothermal est généralement comprise entre 0,5 et 5 heures.

g) réduction de l'oxyde ainsi supporté obtenu à l'étape f), de préférence au moyen d'hydrogène gazeux (étape optionnelle)

Le catalyseur est généralement réduit. Cette étape est de préférence réalisée en présence d'un gaz réducteur, soit *in situ,* c'est-à-dire dans le réacteur où est réalisée la transformation catalytique, soit *ex situ.* De manière préférée, cette étape est effectuée à une température comprise entre 80°C et 180°C, de manière encore plus préférée entre 100°C et 160°C.

La réduction est réalisée en présence d'un gaz réducteur comprenant entre 25 vol% et 100 vol% de dihydrogène, de préférence 100% volume de dihydrogène. Le dihydrogène est éventuellement complété par un gaz inerte pour la réduction, de préférence de l'argon, du diazote ou du méthane.

La réduction comprend généralement une phase de montée en température puis un palier. La durée du palier de réduction est généralement comprise entre 1 et 10 heures, de préférence entre 2 et 8 heures.

La Vitesse Volumétrique Horaire (V.V.H) est généralement comprise entre 150 et 3000, de préférence entre 300 et 1500 litres de gaz réducteur par heure et par litre de catalyseur.

Utilisation du catalyseur

Le catalyseur selon l'invention peut être utilisé dans les procédés d'hydrogénation sélective des composés hydrocarbonés insaturés présents dans les coupes d'hydrocarbures C3 de vapocraquage et/ou de craquage catalytique. Le procédé d'hydrogénation sélective selon l'invention vise à éliminer lesdits hydrocarbures polyinsaturés présents dans ladite charge à hydrogéner sans hydrogéner les hydrocarbures mono-insaturés. Plus particulièrement, le procédé d'hydrogénation sélective selon l'invention vise à hydrogéner sélectivement le propadiène et le méthylacétylène.

Ainsi, par exemple, la coupe C3 de vapocraquage peut avoir la composition moyenne suivante : de l'ordre de 90% poids propylène, de l'ordre de 3 à 8% poids de propadiène et méthylacétylène, le reste étant essentiellement du propane. Dans certaines coupes C3, entre 0,1 et 2% poids de C2 et de C4 peut aussi être présent. Les spécifications concernant les concentrations de ces composés polyinsaturés pour les unités de pétrochimie et de polymérisation sont très basses : 20-30 ppm poids de MAPD (méthylacétylène et propadiène) et propylène supérieure à 95% poids pour le propylène qualité chimique et moins de 10 ppm poids voire jusqu'à 1 ppm poids pour la qualité « polymérisation » et propylène supérieure à 99% poids.

La mise en œuvre technologique du procédé d'hydrogénation sélective est par exemple réalisée par injection, en courant ascendant ou descendant, de la charge d'hydrocarbures polyinsaturés et de l'hydrogène dans au moins un réacteur à lit fixe. Ledit réacteur peut être de type isotherme ou de type adiabatique. Un réacteur adiabatique est préféré. La charge d'hydrocarbures polyinsaturés peut avantageusement être diluée par une ou plusieurs ré-injection(s) de l'effluent, issu dudit réacteur où se produit la réaction d'hydrogénation sélective, en divers points du réacteur, situés entre l'entrée et la sortie du réacteur afin de limiter le gradient de température dans le réacteur. La mise en œuvre technologique du procédé d'hydrogénation sélective selon l'invention peut également être avantageusement réalisée par l'implantation d'au moins dudit catalyseur supporté dans une colonne de distillation réactive ou dans des réacteurs - échangeurs. Le flux d'hydrogène peut être introduit en même temps que la charge à hydrogéner et/ou en un ou plusieurs points différents du réacteur.

L'hydrogénation sélective des coupes C3 peut être réalisée en phase gazeuse ou en phase liquide, de préférence en phase liquide. Une réaction en phase liquide permet d'abaisser le coût énergétique et d'augmenter la durée de cycle du catalyseur.

D'une manière générale, l'hydrogénation sélective des coupes C3 s'effectue à une température comprise entre 0 et 300°C, de préférence entre 20 et 150°C, à une pression comprise entre 0,1 et 10 MPa, de préférence entre 0,5 et 5 MPa, à un ratio molaire hydrogène/(composés polyinsaturés à hydrogéner) entre 0,1 et 10 et à une vitesse volumique horaire V.V.H. (définie comme le rapport du débit volumique de charge sur le volume du catalyseur) comprise entre 0,1 et 200 h$^{-1}$ pour un procédé réalisé en phase liquide, à un ratio molaire hydrogène/(composés polyinsaturés à hydrogéner) entre 0,5 et 1000 pour un procédé en phase gazeuse et à une vitesse volumique horaire V.V.H. entre 100 et 40000 h$^{-1}$ pour un procédé réalisé en phase gazeuse.

Exemples

Les exemples présentés ci-dessous visent à démontrer l'amélioration de l'activité catalytique en hydrogénation sélective des catalyseurs selon l'invention. Les exemples 1 à 4 concernent des procédés de préparation de catalyseurs non conformes à l'invention (catalyseurs C1 à C4) et les exemples 5 à 7 concernent des procédés de préparation d'un catalyseur selon l'invention (catalyseurs C5 à C7).

L'exemple 8 concerne la mesure de la dispersion métallique D des catalyseurs C1 à C7. L'exemple 9 concerne l'application de ces catalyseurs dans une réaction d'hydrogénation sélective d'une coupe C3.

Ces exemples sont présentés à titre d'illustration et ne limitent en aucune manière la portée de l'invention.

Exemple 1 : préparation d'un catalyseur C1 non conforme

Une solution aqueuse de chloropalladate de sodium $Na_2PdCl_4$ est préparée à 25°C par dilution de 3,5 g d'une solution chloropalladate de sodium $Na_2PdCl_4$ contenant 8,5 % poids de palladium, avec de l'eau déminéralisée à un volume qui correspond au volume poreux du support alumine.

Cette solution est ensuite imprégnée sur 100 grammes d'une alumine de type $\delta$-$\gamma$- $Al_2O_3$ dont la $S_{BET}$ est de 130 $m^2$/g. Cette alumine est sous forme de billes dont le diamètre moyen est de 3 mm.

Le catalyseur C1 obtenu est séché sous air à 120°C, puis est calciné pendant 2 heures à 450°C sous un flux d'air de combustion avec une V.V.H. de 500 litres d'air de combustion par litre de catalyseur et par heure. L'air de combustion contient environ 60 g d'eau par kg d'air. Le catalyseur C1 contient 0,3 % en poids de palladium par rapport au poids total du catalyseur.

La caractérisation du catalyseur par microsonde de Castaing conduit à 80 % du Pd situé dans une croûte d'épaisseur 250 $\mu$m.

Avant la chimisorption de CO et le test catalytique, le catalyseur est finalement réduit sous un flux d'hydrogène de V.V.H. 500 litres d'hydrogène par litre de catalyseur et par heure, avec une vitesse de montée en température de 300°C/h et un palier à 150°C pendant 2 heures.

Exemple 2 : préparation d'un catalyseur C2 non conforme

Une solution aqueuse de chloropalladate de sodium $Na_2PdCl_4$ est préparée à 25°C par dilution de 3,5 g d'une solution chloropalladate de sodium $Na_2PdCl_4$est contenant 8,5 % poids de palladium, avec de l'eau déminéralisée à un volume qui correspond au volume poreux du support alumine.

Cette solution est ensuite imprégnée sur 100 grammes d'une alumine de type $\alpha$-$\theta$-$Al_2O_3$ dont la $S_{BET}$ est de 50 $m^2$/g. Cette alumine est sous forme de billes dont le diamètre moyen est de 3 mm.

Le catalyseur C2 obtenu est séché sous air à 120°C, puis est calciné pendant 2 heures à 650°C sous un flux d'air sec avec une V.V.H. de 500 litres d'air de combustion par litre de catalyseur et par heure. L'air de combustion contient environ 60 g d'eau par kg d'air.

Le catalyseur C2 contient 0,3 % en poids de palladium par rapport au poids total du catalyseur.

La caractérisation du catalyseur par microsonde de Castaing conduit à 80 % du Pd situé dans une croûte d'épaisseur 350 $\mu$m.

Avant la chimisorption de CO et le test catalytique, le catalyseur est finalement réduit sous un flux d'hydrogène avec une V.V.H. de 500 litres d'hydrogène par litre de catalyseur et par heure, avec une vitesse de montée en température de 300°C/h et un palier à 150°C pendant 2 heures.

Exemple 3 : préparation d'un catalyseur C3 non conforme

Une solution aqueuse d'acétylacétonate de palladium est préparée à 25°C par dilution de 3,5 g d'une solution de d'acétylacétonate de palladium contenant 5,5 % poids de palladium, avec de l'eau déminéralisée à un volume qui correspond au volume poreux du support alumine. Cette solution est ensuite imprégnée sur 100 gramme d'une alumine de type $\alpha$-$\theta$-$Al_2O_3$ dont la $S_{BET}$ est de 50 $m^2$/g. Cette alumine est sous forme de billes dont le diamètre moyen est de 3 mm.

Le catalyseur C3 obtenu est séché sous air à 120°C, puis est calciné pendant 2 heures à 650°C sous un flux d'air de combustion avec une VVH de 500 litres d'air de combustion par litre de catalyseur et par heure. L'air de combustion contient environ 60 g d'eau par kg d'air. Le catalyseur C3 contient 0,3% en poids de palladium par rapport au poids total du catalyseur.

La caractérisation du catalyseur par microsonde de Castaing conduit à 80 % du Pd situé dans une croûte d'épaisseur 450 $\mu$m.

Avant la chimisorption de CO et le test catalytique, le catalyseur est finalement réduit sous un flux d'hydrogène avec une V.V.H. de 500 litres d'hydrogène par litre de catalyseur et par heure, avec une vitesse de montée en température de 300°C/h et un palier à 150°C pendant 2 heures.

Exemple 4 : préparation d'un catalyseur C4 non conforme

Une solution aqueuse de chloropalladate de sodium $Na_2PdCl_4$ est préparée à 25°C par dilution de 3,5 g d'une solution chloropalladate de sodium $Na_2PdCl_4$ est contenant 8,5 % poids de palladium, avec de l'eau déminéralisée à un volume qui correspond au volume poreux du support alumine. Le pH de la solution est 0,7.

Cette solution est ensuite imprégnée sur 100 gramme d'une alumine de type $\alpha$-$\theta$-$Al_2O_3$ dont la $S_{BET}$ est de 50 $m^2$/g. Cette alumine est sous forme de billes dont le diamètre moyen est de 3 mm.

Le catalyseur C4 obtenu est séché sous air à 120°C, puis est calciné pendant 2 heures à 650°C sous un flux d'air de combustion avec une VVH de 500 litres d'air de combustion par litre de catalyseur et par heure. L'air de combustion contient environ 4000 g d'eau par kg d'air. Le catalyseur C4 contient 1,5% en poids de palladium par rapport au poids total du catalyseur.

La caractérisation du catalyseur par microsonde de Castaing conduit à 80 % du Pd situé dans une croûte d'épaisseur 250 $\mu$m.

Avant la chimisorption de CO et le test catalytique, le catalyseur est finalement réduit sous un flux d'hydrogène avec une V.V.H. de 500 litres d'hydrogène par litre de catalyseur et par heure, avec une vitesse de montée en température de 300°C/h et un palier à 150°C pendant 2 heures.

Exemple 5 : préparation d'un catalyseur C5 conforme

Une solution aqueuse de chloropalladate de sodium $Na_2PdCl_4$ est préparée à 25°C par dilution de 3,5 g d'une solution chloropalladate de sodium $Na_2PdCl_4$ est contenant 8,5 % poids de palladium, avec de l'eau déminéralisée à un volume qui correspond au volume poreux du support alumine. Le pH de la solution est 0,7

Cette solution est ensuite imprégnée sur 100 gramme d'une alumine de type $\alpha$-$\theta$-$Al_2O_3$ dont la $S_{BET}$ est de 10 $m^2$/g. Cette alumine est sous forme de billes dont le diamètre moyen est de 3 mm.

Le catalyseur C5 obtenu est séché sous air à 120°C, puis est calciné pendant 2 heures à 650°C sous un flux d'air de combustion avec une V.V.H. de 500 litres d'air de combustion par litre de catalyseur et par heure. L'air de combustion contient environ 4000g d'eau par kg d'air. Le catalyseur C5 contient 0,03% en poids de palladium par rapport au poids total du catalyseur.

La caractérisation du catalyseur par microsonde de Castaing conduit à 80 % du Pd situé dans une croûte d'épaisseur 250 $\mu$m.

Avant la chimisorption de CO et le test catalytique, le catalyseur est finalement réduit sous un flux d'hydrogène avec une V.V.H. de 500 litres d'hydrogène par litre de catalyseur et par heure, avec une vitesse de montée en température de 300°C/h et un palier à 150°C pendant 2 heures.

Exemple 6 : préparation d'un catalyseur C6 conforme

Une solution aqueuse de chloropalladate de sodium $Na_2PdCl_4$ est préparée à 25°C par dilution de 3,5 g d'une solution de chloropalladate de sodium $Na_2PdCl_4$ contenant 8,5 % poids de palladium, avec de l'eau déminéralisée à un volume qui correspond au volume poreux du support alumine.

Cette solution est ensuite imprégnée sur 100 gramme d'une alumine de type $\alpha$-$\theta$-$Al_2O_3$ dont la $S_{BET}$ est de 10 $m^2$/g. Cette alumine est sous forme de billes dont le diamètre moyen est de 3 mm.

Le catalyseur C6 obtenu est séché sous air à 120°C, puis est calciné pendant 2 heures à 650°C sous un flux d'air de combustion avec une V.V.H. de 500 litres d'air de combustion par litre de catalyseur et par heure. L'air de combustion contient environ 3000 g d'eau par kg d'air.

Le catalyseur C6 contient 0,3% en poids de palladium par rapport au poids total du catalyseur.

La caractérisation du catalyseur par microsonde de Castaing conduit à 80 % du Pd situé dans une croûte d'épaisseur 250 $\mu$m.

Avant la chimisorption de CO et le test catalytique, le catalyseur est finalement réduit sous un flux d'hydrogène avec une V.V.H. de 500 litres d'hydrogène par litre de catalyseur et par heure, avec une vitesse de montée en température de 300°C/h et un palier à 150°C pendant 2 heures.

Exemple 7: préparation d'un catalyseur C7 conforme

Une solution aqueuse de nitrate de palladium est préparée à 25°C par dilution de 3,5 g d'une solution de nitrate de palladium contenant 8,5 % poids de palladium, avec de l'eau déminéralisée à un volume qui correspond au volume poreux du support alumine.

Cette solution est ensuite imprégnée sur 100 gramme d'une alumine de type $\alpha$-$\theta$-$Al_2O_3$ dont la $S_{BET}$ est de 10 $m^2$/g. Cette alumine est sous forme de billes dont le diamètre moyen est de 3 mm.

Le catalyseur C7 obtenu est séché sous air à 120°C, puis est calciné pendant 2 heures à 750°C sous un flux d'air de combustion avec une V.V.H. de 500 litres d'air de combustion par litre de catalyseur et par heure. L'air de combustion contient environ 3000 g d'eau par kg d'air, 10 % en volume d'oxygène et 7 % en volume de dioxyde de carbone.

Le catalyseur C7 contient 0,06% en poids de palladium par rapport au poids total du catalyseur.

La caractérisation du catalyseur par microsonde de Castaing conduit à 80 % du Pd situé dans une croûte d'épaisseur 250 $\mu$m.

Avant la chimisorption de CO et le test catalytique, le catalyseur est finalement réduit sous un flux d'hydrogène avec une V.V.H. de 500 litres d'hydrogène par litre de catalyseur et par heure, avec une vitesse de montée en température de 300°C/h et un palier à 150°C pendant 2 heures.

Exemple 8 : Mesure de dispersion métallique D des catalyseurs C1 à C7

Les mesures de dispersions métalliques sont effectuées par chimisorption de monoxyde de carbone CO, sur les catalyseurs C1 à C7 préalablement réduits sous 1 litre d'hydrogène par heure et par gramme de catalyseur, avec une montée en température de 300°C/h et un palier de deux heures à 150°C. Les catalyseurs C1 à C7 sont ensuite balayés pendant 1 heure à 150°C sous hélium puis refroidi jusqu'à 25°C sous hélium.

La chimisorption de CO est réalisée à 25°C en dynamique selon les pratiques habituelles connues de l'homme du métier, elle conduit à un volume de CO chimisorbé, à partir duquel l'homme du métier peut calculer le nombre de molécules de CO chimisorbés.

Un rapport stœchiométrique d'une molécule de CO par atome de Pd de surface est pris en compte pour calculer le nombre d'atomes de Pd de surface. La dispersion est exprimée en % d'atomes de Pd de surface par rapport à la totalité des atomes de Pd présent dans l'échantillon du catalyseur. La dispersion métallique D du palladium des catalyseurs C1 à C7 est présentée dans le tableau 1 ci-après.

Tableau 1: Dispersion métallique D des catalyseurs C1 à C7

| | $S_{BET}$ support ($m^2/g$) | Précurseur | Traitement hydrothermal | Teneur en Pd (% pds) | Dispersion (%) |
|---|---|---|---|---|---|
| C1 (non conforme) | 130 | $Na_2PdCl_4$ | 450°C/60g d'eau* | 0,3 | 33 |
| C2 (non conforme) | 50 | $Na_2PdCl_4$ | 650°C/60g d'eau* | 0,3 | 25 |
| C3 (non conforme) | 50 | acétylacétonate de Pd | 650°C/60g d'eau* | 0,3 | 24 |
| C4 (non conforme) | 50 | $Na_2PdCl_4$ | 650°C/4000g d'eau* | 1,5 | 21 |
| C5 (conforme) | 10 | $Na_2PdCl_4$ | 650°C/4000g d'eau* | 0,03 | 19 |
| C6 (conforme) | 10 | $Na_2PdCl_4$ | 650°C/3000g d'eau* | 0,3 | 12 |
| C7 (conforme) | 10 | $Pd(NO_3)_2$ | 650°C/3000g d'eau* | 0,06 | 15 |
| *par kg d'air sec | | | | | |

Exemple 9 : Utilisation des catalyseurs C1 à C7 pour l'hydrogénation sélective d'une coupe C3 de vapocraquage

Une charge comprenant 92,47% poids de propylène, 4,12% poids de propane, 1,18% poids de méthylacétylène (MA), 1,63% poids de propadiène (PD) est traitée avec les catalyseurs C1 à C7. Avant réaction les catalyseurs d'hydrogénation sélective sont actifs sous un flux d'hydrogène à 160°C pendant 2h.

25 ml de catalyseur sont places dans un réacteur tubulaire en mode up flow. La pression est maintenue à 30 bars (3 MPa) et la température à 27°C. Une vitesse horaire volumique (V.V.H.) de 50h$^{-1}$ est appliquée. Le rapport molaire $H_2$/ MAPD varie entre 0, 5, 10 et 4, 5 mol/mol. La composition de la charge et de l'effluent est mesuré en continue en sortie du réacteur par chromatographie en phase gaz. Les oligomères gazeux sont définis comme les oligomères non piégés par les différents filtres de l'unité et détectés par la colonne chromatographie (composés jusqu'à 6 atomes de carbone).

Les performances des catalyseurs C1 à C7 sont exprimées dans le tableau 2 ci-après.

Tableau 2 : Sélectivité mesurées lors de l'hydrogénation d'une coupe C3

| Catalyseur | Sélectivité en propylène pour une conversion de 90 % du MAPD* (%) | Sélectivité en oligomères gazeux pour une conversion de 90 % du MAPD* (%) |
|---|---|---|
| C1 (non conforme) | 52 | 14 |

(suite)

| Catalyseur | Sélectivité en propylène pour une conversion de 90 % du MAPD* (%) | Sélectivité en oligomères gazeux pour une conversion de 90 % du MAPD* (%) |
|---|---|---|
| C2 (non conforme) | 55 | 14 |
| C3 (non conforme) | 68 | 13 |
| C4 (non conforme) | 60 | 14 |
| C5 (conforme) | 64 | 12 |
| C6 (conforme) | 71 | 5 |
| C7 (conforme) | 70 | 7 |
| *MAPD = méthylacétylène et propadiène | | |

Les catalyseurs C5 à C7 sont conformes à l'invention. Ils présentent à la fois une très bonne sélectivité en propylène et une faible sélectivité en production d'oligomères gazeux du fait d'une faible dispersion du palladium, i.e. en dessous de 20%. En effet, les catalyseurs C5 à C7 produisent très peu de propane et d'oligomères gazeux. Une surfaces spécifique trop élevée et/ou une teneur en palladium trop élevée, ainsi qu'une dispersion métallique en palladium trop élevée conduisent à des catalyseurs peu performants (catalyseurs C1 à C4), produisant trop de propane, composé non désiré pour cette application, et conduisant à des teneurs en oligomères gazeux symptomatiques d'un production d'oligomères et de coke trop important, et donc nuisibles à la durée de vie du catalyseur et à la bonne conduite de l'unité de d'hydrogénation.

## Revendications

1. Catalyseur comprenant une phase active constituée uniquement de palladium, et un support poreux d'alumine dans lequel :

   - la teneur en palladium dans le catalyseur est comprise entre 0,0025 et 1% poids par rapport au poids total du catalyseur ;
   - au moins 80% poids du palladium est réparti dans une croûte à la périphérie du support poreux, l'épaisseur de ladite croûte étant comprise entre 25 et 500 $\mu$m ;
   - la surface spécifique du support poreux, mesurée par physisorption à l'azote, est comprise entre 1 et 50 $m^2/g$ ;
   - la dispersion métallique D du palladium est inférieure à 20% ;
   - le palladium se présente sous la forme de particules de taille moyenne comprise entre 4 à 10 nm ;
   - le volume total poreux du support, mesuré par porosimétrie au mercure, est compris entre 0,1 et 1,5 $cm^3/g$.

2. Catalyseur selon la revendication 1, dans lequel la dispersion métallique D du palladium est inférieure ou égale à 18%.

3. Catalyseur selon les revendications 1 ou 2, dans lequel la teneur en palladium dans le catalyseur est comprise entre 0,025 et 0,8% poids par rapport au poids total du catalyseur.

4. Catalyseur selon l'une quelconque des revendications précédentes, **caractérisé en ce que** la surface spécifique du support poreux, mesurée par physisorption à l'azote, est comprise entre 1 et 40 $m^2/g$.

5. Catalyseur selon l'une quelconque des revendications précédentes, **caractérisé en ce qu'**au moins 80% poids du palladium est réparti dans une croûte à la périphérie du support poreux, l'épaisseur de ladite croûte étant entre 50 et 450 $\mu$m.

6. Catalyseur selon l'une quelconque des revendications précédentes, **caractérisé en ce que** le support poreux comprend entre 0,0050 et 0,25% en poids de soufre par rapport au poids total du catalyseur.

**7.** Procédé de préparation d'un catalyseur selon l'une quelconque des revendications 1 à 6 comprenant les étapes suivantes :

> a) on prépare une solution aqueuse comprenant au moins un sel précurseur du palladium ;
> b) on imprègne ladite solution sur un support poreux d'alumine;
> d) on sèche le précurseur de catalyseur obtenu à l'étape b) à une température comprise entre 70°C et 200°C pendant une durée comprise entre 0,5 et 20 heures ;
> f) on réalise un traitement hydrothermal du catalyseur séché obtenu à l'étape d) à une température comprise entre 600°C et 700°C, sous air, comprenant entre 300 et 4500 grammes d'eau par kg d'air.

**8.** Procédé selon la revendication 7, dans lequel ledit sel précurseur du palladium est choisi parmi le chloropalladate de sodium ou le nitrate de palladium.

**9.** Procédé selon les revendications 7 ou 8, dans lequel à l'étape b) on imprègne ladite solution sur un support poreux par imprégnation à sec.

**10.** Procédé selon l'une quelconque des revendications 7 à 9, dans lequel à l'étape f) on réalise un traitement hydrothermal du catalyseur séché obtenu à l'étape d) à une température comprise entre 600°C et 700°C, sous air, comprenant entre 500 et 4000 grammes d'eau par kg d'air.

**11.** Procédé selon l'une quelconque des revendications 7 à 10, dans lequel on soumet le support poreux imprégné obtenu à l'étape b) à une étape c) de maturation afin d'obtenir un précurseur de catalyseur.

**12.** Procédé selon l'une quelconque des revendications 7 à 11, dans lequel on réalise une étape e) de calcination du catalyseur séché obtenu à l'étape d) à une température comprise entre 250 et 900°C.

**13.** Procédé selon l'une quelconque des revendications 7 à 12, dans lequel on soumet le catalyseur obtenu à l'issue de l'étape f) à une étape g) de traitement réducteur par mise en contact avec un gaz réducteur.

**14.** Procédé d'hydrogénation sélective comprenant la mise en contact d'une coupe C3 de vapocraquage et/ou de craquage catalytique avec le catalyseur selon l'une quelconque des revendications 1 à 6 ou préparé selon l'une quelconque des revendications 7 à 13, dans lequel la température est comprise entre 0 et 300°C, à une pression comprise entre 0,1 et 10 MPa, à un ratio molaire hydrogène/(composés polyinsaturés à hydrogéner) entre 0,1 et 10 et à une vitesse volumique horaire V.V.H. comprise entre 0,1 et 200 $h^{-1}$ pour un procédé réalisé en phase liquide, à un ratio molaire hydrogène/(composés polyinsaturés à hydrogéner) entre 0,5 et 1000 et à une vitesse volumique horaire V.V.H. entre 100 et 40000 $h^{-1}$ pour un procédé réalisé en phase gazeuse.

**Patentansprüche**

**1.** Katalysator, umfassend eine aktive Phase, die ausschließlich aus Palladium besteht, und einen porösen Aluminiumoxidträger, wobei:

> - der Palladiumgehalt in dem Katalysator zwischen 0,0025 und 1 Gew.-%, bezogen auf das Gesamtgewicht des Katalysators, liegt;
> - mindestens 80 Gew.-% des Palladiums in einer Kruste an der Peripherie des porösen Trägers verteilt sind, wobei die Dicke der Kruste zwischen 25 und 500 μm liegt;
> - die durch Stickstoff-Physisorption gemessene spezifische Oberfläche des porösen Trägers zwischen 1 und 50 $m^2$/g liegt;
> - die Metalldispersion D des Palladiums weniger als 20 % beträgt;
> - das Palladium in Form von Teilchen mit einer mittleren Größe zwischen 4 bis 10 nm vorliegt,
> - das durch Quecksilber-Porosimetrie gemessene Gesamtporenvolumen des Trägers zwischen 0,1 und 1,5 $cm^3$ liegt.

**2.** Katalysator nach Anspruch 1, wobei die Metalldispersion D des Palladiums kleiner oder gleich 18 % ist.

**3.** Katalysator nach Anspruch 1 oder 2, wobei der Palladiumgehalt in dem Katalysator zwischen 0,025 und 0,8 Gew.-%, bezogen auf das Gesamtgewicht des Katalysators, liegt.

4. Katalysator nach einem der vorhergehenden Ansprüche, **dadurch gekennzeichnet, dass** die durch Stickstoff-Physisorption gemessene spezifische Oberfläche des porösen Trägers zwischen 1 und 40 m$^2$/g liegt.

5. Katalysator nach einem der vorhergehenden Ansprüche, **dadurch gekennzeichnet, dass** mindestens 80 Gew.-% des Palladiums in einer Kruste an der Peripherie des porösen Trägers verteilt sind, wobei die Dicke der Kruste zwischen 50 und 450 μm liegt.

6. Katalysator nach einem der vorhergehenden Ansprüche, **dadurch gekennzeichnet, dass** der poröse Träger zwischen 0,0050 und 0,25 Gew.-% Schwefel, bezogen auf das Gesamtgewicht des Katalysators, umfasst.

7. Verfahren zur Herstellung eines Katalysators nach einem der Ansprüche 1 bis 6, das die folgenden Schritte umfasst:

a) man stellt eine wässrige Lösung her, die mindestens ein Vorläufersalz von Palladium umfasst;
b) man imprägniert die Lösung auf einen porösen Aluminiumoxidträger;
d) man trocknet den in Schritt b) erhaltenen Katalysator bei einer Temperatur zwischen 70 °C und 200 °C über einen Zeitraum zwischen 0,5 und 20 Stunden;
f) man führt eine Hydrothermalbehandlung des in Schritt d) erhaltenen getrockneten Katalysators bei einer Temperatur zwischen 600 °C und 700 °C an Luft, die zwischen 300 und 4500 Gramm Wasser pro kg Luft umfasst, durch.

8. Verfahren nach Anspruch 7, wobei das Vorläufersalz von Palladium aus Natriumchloropalladat oder Palladiumnitrat ausgewählt wird.

9. Verfahren nach Anspruch 7 oder 8, wobei man in Schritt b) die Lösung durch Trockenimprägnierung auf einen porösen Träger imprägniert.

10. Verfahren nach einem der Ansprüche 7 bis 9, wobei man in Schritt f) eine Hydrothermalbehandlung des in Schritt d) erhaltenen getrockneten Katalysators bei einer Temperatur zwischen 600 °C und 700 °C an Luft, die zwischen 500 und 4000 Gramm Wasser pro kg Luft umfasst, durchführt.

11. Verfahren nach einem der Ansprüche 7 bis 10, wobei man den in Schritt b) erhaltenen imprägnierten porösen Träger einem Schritt c) der Reifung zum Erhalt eines Katalysatorvorläufers unterwirft.

12. Verfahren nach einem der Ansprüche 7 bis 11, wobei man einen Schritt e) der Calcinierung des in Schritt d) erhaltenen getrockneten Katalysators bei einer Temperatur zwischen 250 und 900 °C durchführt.

13. Verfahren nach einem der Ansprüche 7 bis 12, wobei man den am Ende von Schritt f) erhaltenen Katalysator einem Schritt g) der Reduktionsbehandlung durch Inkontaktbringen mit einem Reduktionsgas unterwirft.

14. Verfahren zur selektiven Hydrierung, umfassend das Inkontaktbringen eines C3-Schnitts aus dem Dampfcracken und/oder katalytischen Cracken mit dem Katalysator nach einem der Ansprüche 1 bis 6 oder dem nach einem der Ansprüche 7 bis 13 hergestellten Katalysator, wobei die Temperatur zwischen 0 und 300 °C liegt, bei einem Druck zwischen 0,1 und 10 MPa, einem Molverhältnis von Wasserstoff zu mehrfach ungesättigten Verbindungen, die zu hydrieren sind, zwischen 0,1 und 10 und einer Katalysatorbelastung VVH zwischen 0,1 und 200 h$^{-1}$ für ein in der Flüssigphase durchgeführtes Verfahren oder bei einem Molverhältnis von Wasserstoff zu mehrfach ungesättigten Verbindungen, die zu hydrieren sind, zwischen 0,5 und 1000 und einer Katalysatorbelastung VVH zwischen 100 und 40.000 h$^{-1}$ für ein in der Gasphase durchgeführtes Verfahren.

**Claims**

1. Catalyst comprising an active phase consisting solely of palladium, and a porous alumina support, in which:

- the palladium content in the catalyst is between 0.0025% and 1% by weight relative to the total weight of the catalyst;
- at least 80% by weight of the palladium is distributed in a crust at the periphery of the porous support, the thickness of said crust being between 25 and 500 μm;
- the specific surface area of the porous support, measured by nitrogen physisorption, is between 1 and 50 m$^2$/g;

- the metallic dispersion D of the palladium is less than 20%;
- the palladium is in the form of particles having a mean size of between 4 and 10 nm;
- the total pore volume of the support, measured by mercury porosimetry, is between 0.1 and 1.5 $cm^3$/g.

2. Catalyst according to Claim 1, in which the metallic dispersion D of the palladium is less than or equal to 18%.

3. Catalyst according to Claim 1 or Claim 2, in which the palladium content in the catalyst is between 0.025% and 0.8% by weight relative to the total weight of the catalyst.

4. Catalyst according to any one of the preceding claims, **characterized in that** the specific surface area of the porous support, measured by nitrogen physisorption, is between 1 and 40 $m^2$/g.

5. Catalyst according to any one of the preceding claims, **characterized in that** at least 80% by weight of the palladium is distributed in a crust at the periphery of the porous support, the thickness of said crust being between 50 and 450 $\mu$m.

6. Catalyst according to any one of the preceding claims, **characterized in that** the porous support comprises between 0.0050% and 0.25% by weight of sulfur relative to the total weight of the catalyst.

7. Process for preparing a catalyst according to any one of Claims 1 to 6, comprising the following steps:

   a) preparing an aqueous solution comprising at least one palladium precursor salt;
   b) impregnating said solution on a porous alumina support;
   d) drying the catalyst precursor obtained in step b) at a temperature between 70°C and 200°C for a duration of between 0.5 and 20 hours;
   f) carrying out a hydrothermal treatment of the dried catalyst obtained in step d) at a temperature of between 600°C and 700°C, in air, comprising between 300 and 4500 grams of water per kg of air.

8. Process according to Claim 7, in which said palladium precursor salt is chosen from sodium chloropalladate and palladium nitrate.

9. Process according to Claim 7 or Claim 8, in which, in step b), said solution is impregnated on a porous support by dry impregnation.

10. Process according to any one of Claims 7 to 9, in which, in step f), a hydrothermal treatment of the dried catalyst obtained in step d) is carried out at a temperature between 600°C and 700°C, in air comprising between 500 and to 4000 grams of water per kg of air.

11. Process according to any one of Claims 7 to 10, in which the impregnated porous support obtained in step b) is subjected to a maturing step c) in order to obtain a catalyst precursor.

12. Process according to any one of Claims 7 to 11, in which a step e) of calcining the dried catalyst obtained in step d) is carried out at a temperature of between 250°C and 900°C.

13. Process according to any one of Claims 7 to 12, in which the catalyst obtained at the end of step f) is subjected to a step g) of reducing treatment by bringing into contact with a reducing gas

14. Selective hydrogenation process, comprising bringing a steam cracking and/or catalytic cracking C3 fraction into contact with the catalyst according to any one of Claims 1 to 6 or prepared according to any one of Claims 7 to 13, in which the temperature is between 0°C and 300°C, at a pressure of between 0.1 to 10 MPa, with a hydrogen/(polyunsaturated compounds to be hydrogenated) molar ratio of between 0.1 and 10 and at an hourly space velocity, HSV, of between 0.1 and 200 $h^{-1}$ for a process carried out in the liquid phase, a hydrogen/(polyunsaturated compounds to be hydrogenated) molar ratio of between 0.5 and 1000 and at an hourly space velocity, HSV, of between 100 and 40 000 $h^{-1}$ for a process carried out in the gas phase.

**RÉFÉRENCES CITÉES DANS LA DESCRIPTION**

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- US 2006025302 A
- FR 2882531
- FR 2991197
- FR 2458524
- FR 2070995
- US 2005137433 A

**Littérature non-brevet citée dans la description**

- CRC Handbook of Chemistry and Physics. 2000
- **R. VAN HARDEVELD ; F. HARTOG.** The statistics of surface atoms and surface sites on metal crystals. *Surface Science,* 1969, vol. 15, 189-230
- **L. SORBIER et al.** Measurement of palladium crust thickness on catalyst by EPMA. *Materials Science and Engineering,* 2012, 32
- Analyse physico-chimique des catalyseurs industriels. 2001
- **ROUQUEROL F. ; ROUQUEROL J. ; SINGH K.** Adsorption by Powders & Porous Solids: Principle, methodology and applications. Academic Press, 1999